# EUROPEAN PATENT APPLICATION

(11) **EP 2 886 156 A1**
(43) Date of publication of application: **24.06.2015**
(21) Application number: 14380037.3
(22) Date of filing: 25.11.2014
(51) Int. Cl.: A61N 1/20, A61N 1/16, A61N 1/14

(54) **Electromagnetic wave decontaminating device for persons**

(30) Priority: 26.11.2013 ES 201301012 U
(71) Applicant: Bohorquez Vega, Maria Dolores, 41089 Dos Hermanas (Sevilla) (ES)
(72) Inventor: Bohorquez Vega, Maria Dolores, 41089 Dos Hermanas (Sevilla) (ES)

(57) **Abstract**

The device comprises a voltage converter that receives altern voltage through a connection to the grid (4) with ground connection (5) and transform it at the exit of the converter into direct voltage, a first conductor (2) electrically connected to the positive pole (6) at the exit of the converter and a second conductor (3) electrically connected to the ground connection (5) of the grid (4), so that, a user who connects to the first conductor (2) and to the second conductor (3), when turning the voltage converter on, receives an electric current across his body, that produces the discharge of residual electromagnetic energy stored in the body.

## Description

### OBJECT OF THE INVENTION

The current invention refers to an electromagnetic wave decontaminating device that, when a user is connected to it by two conductors, produces an electric current that goes over the body of the user between both conductors and removes a big spectrum of electromagnetic waves that residually are stored at the user and remain permanent in them.

It has a special application in the industry related with personal well-being.

### TECHNICAL PROBLEM TO SOLVE AND BACKGROUND OF THE INVENTION

The current electronic era, where technological development is focused on improving people's terms of life, making day-to-day more comfortable, makes available to the consumer a never-ending series of electronic devices that incorporate, not only the devices themselves, but also the way they are connected, a global trend being to avoid physical connections between different devices. This implies that connections take place as electromagnetic waves, devices connecting via wi-fi, bluetooth or any other similar kind of waves being used increasingly. The most usual situations are those concerning to the broadcasting of mobile telephone signals or the internet and the connections performed to connect sound devices, like speakers or amplifiers to other devices such as televisions, computers or even small sound devices like MP3 readers.

In this way, the human being increasingly demands a coverage of signals that covers the whole of the space he moves around, including subways and places of difficult access but frequent use, as, for instance, the underground, underground parking or even shopping centers under the level of the ground. To receive these signals, the installation of huge antenna invading the space with electromagnetic waves is needed.

Together to this technological development, studies on how these waves influence our organism have been performed, as the fact that they are unnoticeable for people does not mean they are harmless. Actually, electromagnetic waves distort electric pulses used by the brain to send information to the different organs of the body, causing this information to have an interference that falsifies or reduces the right pulses and making the organs of the body not to receive the right information they need to work properly.

It has been detected that electromagnetic waves progressively install in the human body and remain permanently, causing a series of symptom that may arise either independently or some of them simultaneously. Among these symptoms the following can be highlighted as the most usual:
- General malaise caused by weariness, pain or depression.
- Generalized pain, with the sensation of not being able to specify a particular zone of the body.
- Localized pain, specially in very specific parts of the body, as the head, limbs, legs, arms, low back, waist, back, vertebras or fingers, among others.
- Weariness and chronic fatigue, with the sensation of a generalized exhaustion during the whole day, from the morning, affecting so usual acts as getting down and standing up, going up stairs, getting a baby in arms, talking to people, dressing kids, doing the shopping, washing the hair or any home tasks, among others, requiring a special effort that consumes the small energy remaining in the body.
- Lapse of concentration, lack of reflex, unsafe sensation when driving, lack of capacity not to lose the thread of conversations or meetings, continuous oversights about usual acts, etc.
- Insomnia.
- Nervousness and stress.
- Irritability, lack of control, easy irritation, no capacity to support jokes and other typical behavior of children.
- Lack of sexual appetite.
- Apathy, indolence and lack of motivation in general.
- Depression.
- Other affections or symptoms which cause neither has a pathological origin nor can be explained from the medical point of view even after performing all kind of medical tests, as blood tests, resonances, radiographs, ultrasounds, tacs, etc.

A diversity of devices and methods focused on the eradication of this kind of pains permanent or regular, are known in the current state of the art, these methods being either considered as therapeutic treatment or just as momentary stimulators temporarily soothing the annoying sensation.

These devices or treatment methods are mainly based on the application of magnetic fields that, when oriented the right way, produce benefits in the body of the user. Other devices are developed from electric currents going over parts of the human body through electric connectors located in specific places.

Document US2005149124A describes a method for the treatment of an infected area of a person where the infected area is exposed to a water solution and an electric current is applied to the water solution in order to treat the infected area.

Document US7291162A describes a method to restore the inner energy flux in the human body of a patient to a natural tract of the body, consisting in locating a series of patches electrically connected with wires. These patches define parts of the body where energy jumps take place through which the electric energy of the body is restored to its natural tract. In one embodiment, a first patch is located on the navel of the body, and another two patches under each of the collar bones in a determined position, all patches having electric connectors in direct connection with the body of the patient. Through the electric connection of the patch on the navel with each one of the other two patches on the collar bones, the position of the three patches is held for some minutes, letting the transfer of electric energy flux in the body t its natural tract.

It has to be said that methods for curing the pains above described have been spreading in their use to be applied subsequently in other kind of similar pains where, even if the cause was not the same, symptoms were and, therefore, a similar way of palliative could be considered. The field where they have probably most widely spread has been fibromyalgia, as no eradication treatment seems to have been found for the pains it causes, but only for temporarily palliating them.

Anyway, treatments currently followed by patients who suffer these pains either have a very mild healing effect or are based on visiting important medical facilities where the cost of the sessions makes it unaffordable to most of the public.

The device of the current invention solves this problem with the application on the body of the user of a low voltage electric current, treating a usual problem for the patients suffering this kind of pains in a very economic way, very affordable and that can be performed so often as demanded, as there are no contraindications or side effects. Therefore, the problem created by the above described remaining charges on the bodies of the users is solved with the current invention, not knowing to the moment the existence in the current state of the art of a device with a similar performance.

### DESCRIPTION OF THE INVENTION

The current invention describes a electromagnetic wave decontaminating device for human beings, preferably persons. The device comprises a voltage converter that receives altern voltage via a three-core wire that is plugged to the grid in a socket with ground connection and transforms it into direct voltage at the converter outlet, and two conductors, the first of them connected to the positive pole at the converter outlet and the second conductor connected to ground. It is not needed, but it is convenient for the converter to have a switch to connect and disconnect the voltage of the connector, together with a LED indicator to indicate whether the device is on or off.

When connecting the decontaminating device to the body of an user via the two conductors, once the converter is switched on, an electric circuit is closed where a current is generated from the positive pole at the converter outlet that sweeps along residual and parasite currents that are evacuated along the ground connection of the circuit, producing the discharge of the residual and parasite electromagnetic energy stored in the body of the user.

The tests performed have shown that the most effective discharge of this residual electromagnetic energy is by closing the circuit with the ground connection and not with the negative pole at the converter outlet, which would limit the discharge or would do it slower.

The first conductor is formed by a body in a material with a high electric conductivity, typically metallic, which is located in one hand of the user to be decontaminated. The geometry of the body of the first conductor is preferably a cylinder or similar.

The second conductor is formed by a body in a material with a high electric conductivity, typically metallic, on which a foot of the patient to be decontaminated is located. The geometry of the second conductor is preferably a plate or similar.

Other embodiments imply that the first conductor might be held by the two hands of the user or that the two feet of the user are located on the second conductor.

The target of these embodiments is to cover the longest trajectory performed by the electric current in the body of the user, so that the circuit should have a longitudinal shape, a Y shape or an X shape, depending on the number of limbs taking part in the contact.

### BRIEF DESCRIPTION OF THE FIGURES

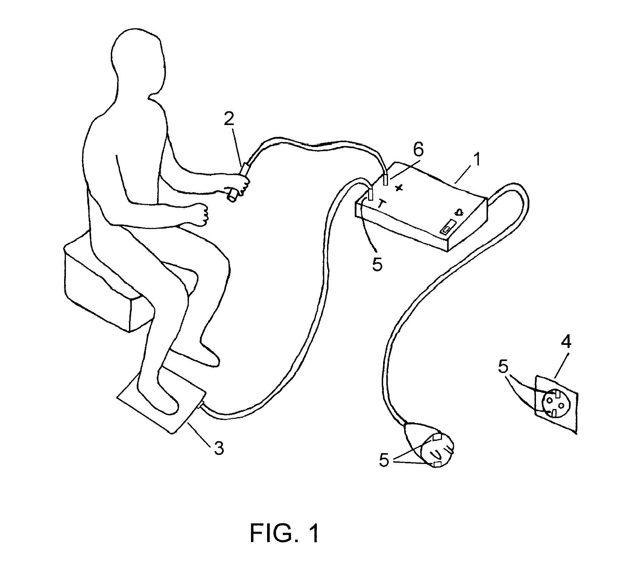

In order to complete the invention described and focused to help for a better understanding of the invention according to a preferred embodiment, following is a figure which, in an illustrative way and not restricting, figure 1 has been represented, depicturing a perspective view of the device of the invention connected to the body of an user through a hand and a foot.

Following is a list of the references used in the figure:
1. Decontaminating device.
2. First conductor.
3. Second conductor.
4. Grid.
5. Ground connection.
6. Positive pole at the converter outlet.

### DESCRIPTION OF A PREFERRED EMBODIMENT.

The present invention refers to an electromagnetic wave decontaminating device for persons.

One of the main components of the device (1) is a voltage converter device that transforms the altern voltage from the grid (4) at the converter inlet into a direct voltage at the converter outlet, not represented in the figure.

A main requirement of the invention is that the electric socket where the electric converter is plugged to the grid must have ground connection (5).

So, a three-core wire, one of whose ends is connected to the voltage converter and whose other end finishes in a plug to connect to the grid (4) with ground connection (5), extends from the device (1). The ground wire of this three-core wire is not connected to the converter but it extends to the housing of the device (1) finishing in a first socket where the second conductor is connected with a wire connected to it or just crossing the housing to directly connect to the second conductor (3).

In the same way, at the converter outlet, in the zone of direct voltage, the positive pole (6) is connected to a wire that extends either up to the housing of the device (1) finishing in a second socket where the first conductor (3) is plugged with a wire connected to it or just crossing the housing to directly connect to the first conductor (2).

The value of the altern voltage in the converter inlet is designed for the voltage values of the Spanish grid, 230V, although this value does not affect the invention, and is just considered as a feature of design, with the possibility to be 230V, 115V or any other value.

The value of the direct voltage at the converter outlet is preferably between 5 and 50V, although not limited to this range.

As depictured in figure 1, besides the three-core wire finishing in a plug to connect to the grid (4), a wire connected to a first conductor (2) and another wire connected to a second conductor (3) extend from the device (1). The first conductor (2) is connected to the positive pole (6) at the voltage converter outlet. The second conductor (3) is connected to the ground connection (5).

When a user starts a decontaminating treatment, the user must connect to the device (1). So, he must close the electric circuit with his body, contacting with both conductors (2, 3). The user connects so that a part of his body contacts the first conductor (2) and another part of his body contacts the second conductor (3). When turning the device (1) on, the electric current going over the body of the user sweeps along residual and permanent parasite currents in the body, causing the electromagnetic decontamination. For this reason, a recommendation is to locate the first conductor (2) in the body of the user as far as possible from the second conductor (3), trying to make the circuit along the body of the user as long as possible between both conductors (2, 3). This way, the efficiency of the process will be most effective and the maximum effect of electromagnetic decontamination will be achieved.

After performing several tests where different locations for the connectors (2, 3) have been tried, the conclusion was that the most effective locations are the ends of the limbs. Actually, the most effective locations are those where one of the conductors (2, 3) is held with the hand and the other conductor (2, 3) is located in the foot. Based on this, there are different embodiments consisting on the place where the conductors (2, 3) are located. These may be the following:
- grab the first conductor (2) with one hand and lean one foot, preferably the foot on the opposite side of the body, on the second conductor (3),
- grab the first conductor (2) with both hands and lean one foot on the second conductor (3),
- grab the first conductor (2) with one hand and lean both feet on the second conductor (3),
- grab the first conductor (2) with both hands and lean both feet on the second conductor (3).

In search for the top efficiency of the process, conductors (2, 3) are designed in shapes ergonomically compatible with the shapes of the body they will host. So, the first conductor (2) has preferably the shape of a cylinder or similar, to be grabbed by the hand of the user. The second conductor (3) has preferably the shape of a plate or flat surface and it is there where the bare foot of the user is to be located. So, in a decontaminating session, the user will hold the first conductor (2) with one or with both hands, closing them around it, in order to have as much contact as possible. The foot or the feet, must remain leaning on the plate forming the second conductor (3). It is important to reiterate that the hands so as the feet contacting the conductors (2, 3) must remain bare during the session, so that the intensity of the electrical transmission is as high as possible.

Additionally, the materials the conductors (2, 3) are made of are high electrical conductivity materials, preferably metallic as, for instance, copper or aluminum.

The tests performed with prototypes of the device have determined that a decontaminating session must long between 5 and 15 minutes, depending on the volume of the body of the patient to decontaminate, the residual electromagnetic charge in the body of the patient and the frequency of use of the decontaminating sessions.

It must be highlighted that the current invention must not be limited to the embodiment above described. Other configurations may be performed by the skilled in the art at the sight of the present specification. Therefore, geometries of the conductors (2, 3) so as the parts of the body contacting are merely design options where the major efficiency is pursued, but with the chance to choose other factors, as comfort, effectiveness, mobility or others at the time of implementing the conductors (2, 3).

Therefore, the scope of the invention is defined by the following claims.

## Claims

1. Electromagnetic wave decontaminating device for persons, **characterized in that** it comprises:
- a voltage converter that receives altern voltage from a connection to a grid (4) with ground connection (5) and transforms it at the converter outlet in direct voltage,
- a first conductor (2) electrically connected to the positive pole (6) at the converter outlet, and
- a second conductor (3) electrically connected to the ground connection (5) of the grid (4),
wherein a user who connects to the first connector (2) and to the second connector (3), when turning the voltage converter on, receives an electric current across his body that produces the discharge of residual electromagnetic energy stored in the body.

2. Electromagnetic wave decontaminating device for persons, **characterized in that** the first conductor (2) is formed by a metallic body that is located in one hand or in both hands of the user to decontaminate.

3. Electromagnetic wave decontaminating device for persons, **characterized in that** the second conductor (3) is formed by a metallic body where a foot or both feet of a user to decontaminate is located.

4. Electromagnetic wave decontaminating device for persons, **characterized in that** the first conductor (2) is a cylinder or similar.

5. Electromagnetic wave decontaminating device for persons, **characterized in that** the second conductor (3) is a plate or similar.
